(19) Europäisches Patentamt
European Patent Office
Office européen des brevets

(11) **EP 3 368 759 B1**

(12) **EUROPÄISCHE PATENTSCHRIFT**

(45) Veröffentlichungstag und Bekanntmachung des Hinweises auf die Patenterteilung:
**06.04.2022 Patentblatt 2022/14**

(21) Anmeldenummer: **16785423.1**

(22) Anmeldetag: **18.10.2016**

(51) Internationale Patentklassifikation (IPC):
***F02D 41/14*** *(2006.01)*

(52) Gemeinsame Patentklassifikation (CPC):
**G01N 27/4175; F02D 41/144; F02D 41/1454; G01N 33/0036;** F02D 41/0072; F02D 41/0077; F02D 41/123; F02D 41/1406; F02D 41/1456

(86) Internationale Anmeldenummer:
**PCT/EP2016/074971**

(87) Internationale Veröffentlichungsnummer:
**WO 2017/071989 (04.05.2017 Gazette 2017/18)**

(54) **VERFAHREN ZUR ERMITTLUNG EINER GASKONZENTRATION IN EINEM MESSGAS MIT EINEM GASSENSOR**

METHOD FOR DETERMINING A GAS CONCENTRATION IN A MEASURING GAS WITH A GAS SENSOR

PROCÉDÉ DE DÉTERMINATION D'UNE CONCENTRATION DE GAZ DANS UN GAZ DE MESURE AU MOYEN D'UN DÉTECTEUR DE GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.10.2015 DE 102015220991**

(43) Veröffentlichungstag der Anmeldung:
**05.09.2018 Patentblatt 2018/36**

(73) Patentinhaber: **Robert Bosch GmbH 70442 Stuttgart (DE)**

(72) Erfinder:
• ELMER, Martin
84166 Adlkofen (DE)
• ZEIN, Thomas
71069 Sindelfingen (DE)
• SCHMIDT, Albrecht
70806 Kornwestheim (DE)

(56) Entgegenhaltungen:
EP-A1- 2 557 418          DE-A1-102006 011 837
DE-U1-202014 002 252      JP-A- H10 176 577
US-A1- 2014 150 760       US-A1- 2015 101 327
US-A1- 2015 267 627

**Beschreibung**

Stand der Technik

Aus dem Stand der Technik sind bereits Gassensoren bekannt und es sind auch Betriebsverfahren zur Ermittlung von Gaskonzentrationen in einem Messgas mit Gassensoren bekannt, die eine Kompensation der Abhängigkeit der von den Gassensoren bereitgestellten Signale von dem Absolutdruck im Messgas vornehmen.

Aus der DE 10 2006 011 837 A1 ist ein Verfahren zur Ermittlung einer Gaskonzentration in einem Messgas mit einem Gassensor bekannt, bei dem bei Vorliegen einer ersten Betriebsart einer Brennkraftmaschine, bei der die Gaskonzentration im Messgas bekannt ist, ein Gaskonzentrationssignal und ein Drucksignal erfasst werden. Es wird dort ferner vorgeschlagen, ausgehend von diesen Signalen einen Kompensationsparameter des Gassensors zu ermitteln. Es ist ferner vorgesehen, den so ermittelten Kompensationsparameter dann in wenigstens einer zweiten Betriebsart der Brennkraftmaschine für die Ermittlung der Gaskonzentration zu berücksichtigen.

Die US 2015/267627 A1 und die US 2014/0150760 A1 offenbaren weitere Verfahren zur Ermittlung einer Gaskonzentration in einem Messgas.

Offenbarung der Erfindung

Erfindungsgemäß wird ein Verfahren zur Ermittlung einer Gaskonzentration in einem Messgas mit einem Gassensors vorgeschlagen, bei dem in einer ersten Betriebsart einer Brennkraftmaschine, bei der die Gaskonzentration im Messgas bekannt ist, eine Vielzahl von Wertepaaren jeweils eines Gaskonzentrationssignals und eines Drucksignals erfasst wird und ausgehend von diesen Wertepaaren ein Kompensationsparameter und ein Skalierungsfaktor des Gassensors ermittelt werden, und bei dem anschließend in einer zweiten Betriebsart der Brennkraftmaschine die Ermittlung einer zu bestimmenden Gaskonzentration erfolgt, auf Basis eines in der zweiten Betriebsart der Brennkraftmaschine gemessenen Gaskonzentrationssignals und unter Berücksichtigung des zuvor ermittelten Kompensationsparameters und des Skalierungsfaktor des Gassensors.

Ferner ist erfindungsgemäß vorgesehen, dass in der ersten Betriebsart der Kompensationsparameter und der Skalierungsfaktor als Lösung von zwei Gleichungen bestimmt werden, wobei in die Gleichungen die Gaskonzentrationssignale und die Drucksignale lediglich mittelbar über Koeffizienten eingehen, die durch Summierung von von den Gaskonzentrationssignalen und den Drucksignalen abhängigen Termen gebildet werden

Eine Vielzahl ist im Rahmen dieser Anmeldung eine natürliche Zahl, die nicht kleiner als 3, insbesondere sogar nicht kleiner als 5 ist. Es ist bevorzugt, dass das Verfahren mit einer hohen Anzahl an Wertepaaren durchgeführt wird, sodass eine Vielzahl im Rahmen dieser Anmeldung insbesondere auch eine natürliche Zahl sein kann, die nicht kleiner als 10 ist.

Die erfindungsgemäße Lösung ist zweckmäßiger als die vorbekannte Lösung, da auf Basis der in der ersten Betriebsart erfassten Wertepaare neben dem Kompensationsparameter gleichzeitig auch ein Skalierungsfaktor des Gassensors ermittelt wird und somit in der zweiten Betriebsart auf Basis eines dann gemessenen Gaskonzentrationssignals eine tatsächliche Gaskonzentration genauer ermittelt werden kann.

Bei dem Kompensationsparameter und dem Skalierungsfaktor handelt es sich insbesondere um Größen, die benutzt werden, um aus den Signalen eines Gassensors (auch: Gaskonzentrationssignalen), zum Beispiel aus einem von dem Gassensor gelieferten Strom oder einer von dem Gassensor gelieferten Spannung oder einer hierzu proportionalen Größe, zum Beispiel einer derart errechneten vermeintlichen Gaskonzentration, auf die tatsächliche Gaskonzentration zu schließen. Diese Größen können in einem Messgas mit einer gegebenen Gaskonzentration für jeden individuellen Gassensor etwas verschieden sein, gemäß Fertigungsstreuungen und oder Alterungsprozessen, denen der individuelle Gassensor unterworfen ist.

Die in der ersten Betriebsart und in der zweiten Betriebsart gemessenen Gaskonzentrationssignale können in einer bevorzugten Weiterbildung von Gassensoren ermittelt werden, die im Ansaugtrakt der Brennkraftmaschine stromabwärts eines Abgasrückführungsventils dem Messgas ausgesetzt sind. Hierbei ist ein Abgasrückführungsventil der Brennkraftmaschine in der ersten Betriebsart vorzugsweise geschlossen, sodass der Gassensor in dieser Betriebsart einem Messgas ausgesetzt ist, bei dem der Anteil der zu ermittelnden Gaskonzentration gleich groß wie in der Umgebungsluft ist, also in der Regel 20,95%.

Bevorzugt werden der Kompensationsparameter und der Skalierungsfaktor in einem Optimierungsverfahren, beispielsweise durch ein Fit-Verfahren bestimmt. Das Optimierungsverfahren minimiert hierfür insbesondere die Summe über alle Wertepaare der Quadrate der Differenzen aus dem jeweiligen Gaskonzentrationssignal und einer vorgegebenen Funktion, die von dem Gaskonzentrationssignal und dem Drucksignal abhängig ist und deren Parameter der Kompensationsparameter und der Skalierungsfaktor sind.

Insbesondere erfolgt die Bestimmung gemäß der folgenden Bedingung:

$$Min_{\{k,m_{adap}\}}\left[\sum_{i=1}^{N}(I_i - I(p_i))^2\right], \text{ wobei } I(p_i) = \frac{I_i}{m_{adap}} \frac{p_i}{k + p_i} \cdot \frac{k + p_0}{p_0},$$

wobei $p_0$ ein Referenzdruck, k der Kompensationsparameter und $m_{adap}$ der Skalierungsfaktor ist, $I_i$ ein Gaskonzentrationssignal und $p_i$ ein Drucksignal und N die Anzahl der erfassten Wertepaare ist.

Anstelle der vorgegebenen Funktion und dem Gaskonzentrationssignal können auch die Kehrwerte dieser Größen verwendet werden.

Der Referenzdruck kann durch den Normaldruck von 1013mbar gegeben sein. Es kann aber auch ein anderer Druck als Referenzdruck verwendet werden, was letztlich ohne wesentliche Auswirkung auf das Gesamtverfahren bleibt, solange in der zweiten Betriebsart die Bestimmung der Gaskonzentration unter Zugrundelegung des gleichen Referenzdrucks erfolgt.

Das oben angegebene Minimierungsproblem kann gelöst werden, indem sowohl die Ableitung der Summe über alle Wertepaare der Quadrate der Differenzen aus dem jeweiligen Gaskonzentrationssignal und der vorgegebenen Funktion nach dem Kompensationsparameter als auch die Ableitung dieser Summe nach dem Skalierungsfaktor zu null gesetzt werden. Alternativ kann auch die Summe über die Quadrate der Differenzen der Kehrwerte des jeweiligen Gaskonzentrationssignal und der vorgegebenen Funktion gebildet werden und die Ableitung dieser Summe nach dem Kompensationsparameter zu null gesetzt wird und zugleich die Ableitung dieser Summe nach dem Skalierungsfaktor zu null gesetzt wird.

Durch identische Umformung ergibt sich dann insbesondere:

$$(-A_1 \cdot B_2 + A_2 \cdot B_1) \cdot k^3 + (-A_1 \cdot B_2 \cdot p_0 + A_2 \cdot B_3 - A_3 \cdot B_2 + A_4 \cdot B_1) \cdot k^2$$
$$+ (-A_3 \cdot B_2 \cdot p_0 + A_4 \cdot B_3 - A_5 \cdot B_2 + A_6 \cdot B_1) \cdot k + (-A_5 \cdot B_2 \cdot p_0 + A_6 \cdot B_3) = 0$$

und

$$m_{adap} = -\frac{B_2(k + p_0)}{B_1 \cdot k + B_3}$$

wobei $p_0$ der Referenzdruck, k der Kompensationsparameter und $m_{adap}$ der Skalierungsfaktor ist und wobei die Koeffizienten insbesondere berechnet werden gemäß:

$$A_1 = \sum_i \frac{p_0^2}{p_i^2}; A_2 = -\sum_i \frac{p_0}{p_i \cdot I_i}; A_3 = 2\sum_i \frac{p_0^2}{p_i}; A_4 = -\sum_i \frac{p_0}{p_i}(p_i + p_0)\frac{1}{I_i}; A_5 = \sum_i p_0^2; A_6 = -\sum_i \frac{p_0^2}{I_i}$$

$$B_1 = \sum_i \frac{p_0^2}{p_i^2}(p_0 - p_i); B_2 = -\sum_i \frac{p_0}{p_i}(p_0 - p_i)\frac{1}{I_i}; B_3 = \sum_i \frac{p_0^2}{p_i}(p_0 - p_i); B_4 = p_0 \cdot B_2$$

Aus der kubischen Gleichung kann der Kompensationsparameter numerisch, beispielsweise mit dem Newtonverfahren, rasch gelöst werden. Mit dem so ermittelten Kompensationsparameter kann dann der Skalierungsfaktor bestimmt werden.

Das Verfahren kann mittels einer elektronischen Steuerungseinheit durchgeführt werden, die ein elektronisches Speichermedium umfasst. Es ist sehr vorteilhaft möglich, in der ersten Betriebsart der Brennkraftmaschine, während die Wertepaare sukzessive erfasst werden, in dem elektronischen Speichermedium lediglich Koeffizienten, zum Beispiel maximal 10 verschiedene Koeffizienten, insbesondere die oben definierten Koeffizienten, zu speichern und diese sukzessive, insbesondere durch Summenbildung, zu aktualisieren. Insbesondere wenn eine sehr hohe Anzahl an Wertepaaren zur Bestimmung des Kompensationsparameters und des Skalierungsfaktor berücksichtigt werden sollen, zum Beispiel mehr als 30 Wertepaare, ergibt sich auf diese Weise eine drastische Reduktion der während des Verfahrens im elektronischen Speichermedium abzulegenden Daten.

Grundsätzlich können sehr viele Wertepaare berücksichtigt werden. Da die Wertepaare ohne weitere Einschränkungen kontinuierlich erfasst werden, können innerhalb kurzer Zeit sogar 1000 und mehr Wertepaare berücksichtigt werden. Da die Koeffizienten sukzessive durch die auf Basis der neuen Wertepaare hinzukommenden Summanden angepasst werden, kann das Verfahren so durchgeführt werden, dass der Einfluss früherer Wertepaare gleichsam fortlaufend mit vorgebbarer Zeitkonstante vergessen wird.

Vorteilhafterweise berücksichtigen die erfassten Wertepaare Drucksignale im gesamten funktionsrelevanten Bereich, der beispielsweise von 500mbar bis 2000mbar oder sogar bis 2500mbar reicht.

In der zweiten Betriebsart der Brennkraftmaschine kann zur Ermittlung der Gaskonzentration insbesondere die Größe $I(p_{meas})$ berechnet werden, die von dem in der zweiten Betriebsart gemessen Gaskonzentrationssignal und dem in der zweiten Betriebsart ermittelten Drucksignal abhängig ist und deren Parameter der Kompensationsparameter und der Skalierungsfaktor sind, insbesondere gemäß dem oben, mit Bezug auf die erste Betriebsart bereits erwähnten vorgegebene Funktion, insbesondere gemäß der Formel

$$I(p_{meas}) = \frac{I_{nom}}{m_{adap}} \frac{p_{meas}}{k + p_{meas}} \cdot \frac{k + p_0}{p_0}$$

wobei $I_{nom}$ das in der zweiten Betriebsart ermittelte Gaskonzentrationssignal und $p_0$ der schon oben erwähnte Referenzdruck, k der Kompensationsparameter und $m_{adap}$ der Skalierungsfaktor ist und $p_{meas}$ das in der zweiten Betriebsart ermittelte Drucksignal ist.

Das in der zweiten Betriebsart ermittelte Drucksignal kann das Ausgangssignal des Gassensors oder eines weiteren Sensors sein, der den Druck am Ort oder in der Nähe des Gassensors zu erfassen vermag.

Es kann sich bei dem in der zweiten Betriebsart ermittelten Drucksignal aber auch um eine Größe handeln, die beispielsweise durch die elektronische Steuerungseinheit mittels eines Abgas-Luftmodells ermittelt wird.

Zeichnungen

Ausführungsbeispiele der Erfindung sind in den Zeichnungen dargestellt und in der nachfolgenden Beschreibung näher erläutert. Es zeigen

Figur 1    schematisch den Aufbau eines Gassensors
Figur 2    erfindungsgemäß ermittelte Wertepaare
Figur 3    weitere erfindungsgemäß ermittelte Wertepaare

Ausführungsbeispiel

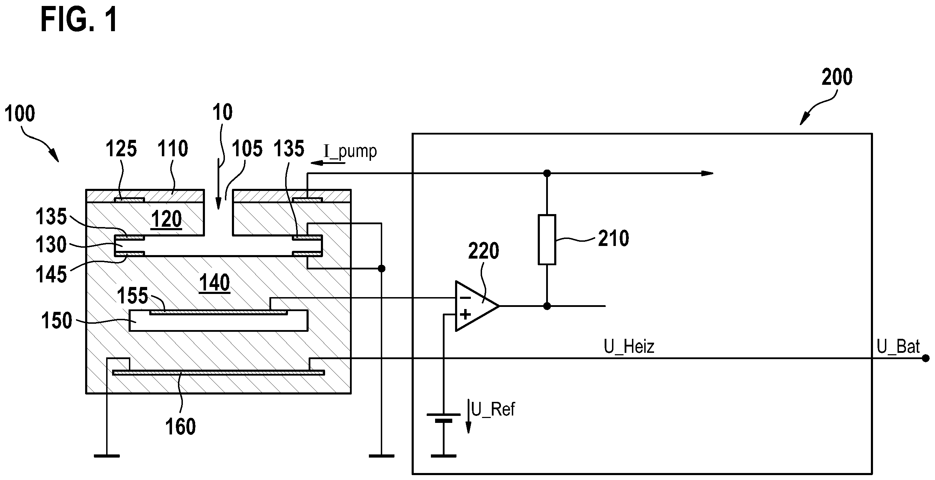

Figur 1 zeigt beispielhaft einen Gassensor 100 zur Bestimmung der Konzentration von Gaskomponenten in einem Gasgemisch mit einer zugehörigen Vorrichtung zur Ansteuerung 200. Der Gassensor ist im vorliegenden Beispiel als Breitband-Lambdasonde ausgestaltet. Sie umfasst im Wesentlichen in einem unteren Bereich eine Heizung 160, in einem mittleren Bereich eine Nernstzelle 140 und in einem oberen Bereich eine Pumpzelle 120. Die Pumpzelle 120 weist in einem zentralen Bereich eine Öffnung 105 auf, durch die Abgas 10 in einen Messraum 130 der Pumpzelle 120 gelangt. An den äußeren Enden des Messraums 130 sind Elektroden 135, 145 angeordnet, wobei die oberen Elektroden 135 der Pumpzelle zugeordnet sind und die Innenpumpelektroden (IPE) 135 bilden, und wobei die unteren Elektroden 145 der Nernstzelle 140 zugeordnet sind und die Nernstelektroden (NE) 145 bilden. Die dem Abgas zugewandte Seite der Pumpzelle 120 weist eine Schutzschicht 110 auf, innerhalb derer eine Außenpumpelektrode (APE) 125 angeordnet ist. Zwischen der Außenpumpelektrode 125 und der Innenpumpelektrode 135 des Messraums 130 erstreckt sich ein Festkörperelektrolyt über den, bei einer an den Elektroden 125, 135 anliegenden Pumpspannung, Sauerstoff in den Messraum 130 transportiert oder aus dem Messraum 130 abtransportiert werden kann.

An die Pumpzelle 120 schließt sich ein weiterer Festkörper an, der die Nernstzelle 140 mit einem Referenzgasraum 150 bildet. Der Referenzgasraum 150 ist in Richtung der Pumpzelle mit einer Referenzelektrode (RE) 155 versehen. Die sich zwischen der Referenzelektrode 155 und der Nernstelektrode 145 im Messraum 130 der Pumpzelle 120 einstellende Spannung entspricht der Nernstspannung. Im weiteren Verlauf der Keramik ist in einem unteren Bereich die Heizung 160 angeordnet.

In dem Referenzgasraum 150 der Nernstzelle 140 wird ein Sauerstoff-Referenzgas vorgehalten. Über einen über die Pumpelektroden 125 und 135 fließenden Pumpstrom wird im Messraum eine Sauerstoffkonzentration eingestellt, die einer "Lambda = 1"-Konzentration in dem Messraum 130 entspricht.

Die Steuerung dieser Ströme und die Auswertung der Nernstspannung übernimmt eine Ansteuerung bzw. ein Steuergerät 200. Einen Operationsverstärker 220 misst hierbei eine an der Referenzelektrode 155 anliegende Nernstspannung und vergleicht diese Spannung mit einer Referenzspannung U_Ref, die typischerweise bei ca. 450 mV liegt. Bei Abweichungen beaufschlagt der Operationsverstärker 220 die Pumpzelle 120 über einen Widerstand 210 und den Pumpelektroden 125, 135 mit einem Pumpstrom.

Das erfindungsgemäße Verfahren kann selbstverständlich auch mit anderen Gassensoren 100 durchgeführt werden, insbesondere wesentlich ist insofern lediglich, dass die Gassensoren 100 Gaskonzentrationssignale liefern, die eine Abhängigkeit vom Absolutdruck des Messgases aufweisen, so wie es beispielsweise auch bei Breitband-Lambdasonden der Fall ist, die lediglich eine einzige elektrochemische Zelle aufweisen, die als Pumpzelle betrieben werden kann, und so wie es auch bei in der Regel drei elektrochemische Zellen aufweisenden NOx-Sensoren der Fall ist.

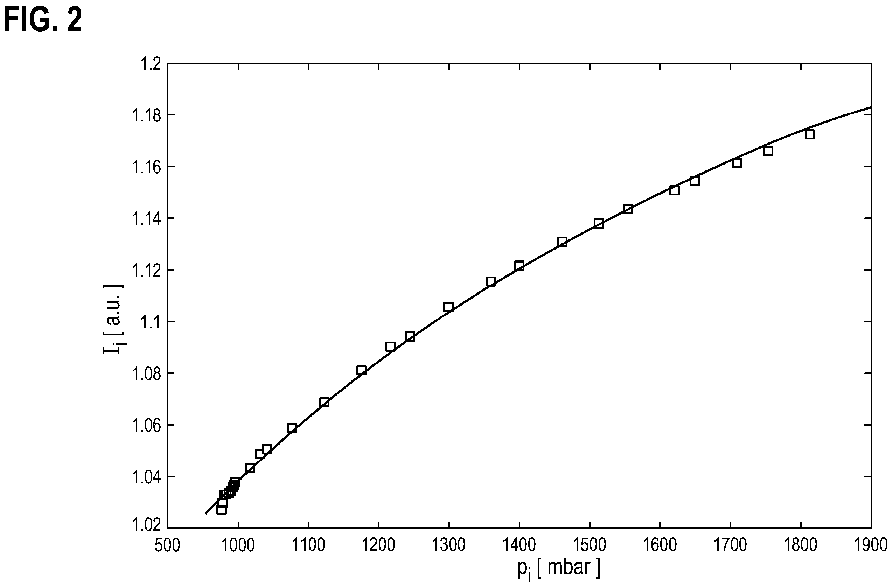

Mit dem Gassensor 100 gemäß Figur 1 wurden in einem Ansaugtrakt einer Brennkraftmaschine stromabwärts eines geschlossenen Abgasrückführungsventils, also in Umgebungsluft, deren Sauerstoffgehalt 20,95 % beträgt, in einem Intervall von Absolutdrücken im Bereich von 950mbar bis 1900mbar 28 Wertepaare $I_i$, $p_i$ aus jeweils einem Wert des Absolutdrucks $p_i$ und des zugehörigen Gaskonzentrationssignal $I_i$ ermittelt. Diese Wertepaare sind in der Figur 2 dargestellt. Alternativ hätten die Wertepaare auch in einem Bereich ermittelt werde können, der von 500mbar bis 2500mbar reicht.

Auf Basis dieser Wertepaare $I_i$, $p_i$ wurden zunächst sukzessive die Koeffizienten $A_1$ - $A_6$, $B_1$ - $B_4$ gemäß der Summenformeln

$$A_1 = \sum_i \frac{p_0^2}{p_i^2}; A_2 = -\sum_i \frac{p_0}{p_i \cdot I_i}; A_3 = 2\sum_i \frac{p_0^2}{p_i}; A_4 = -\sum_i \frac{p_0}{p_i}(p_i + p_0)\frac{1}{I_i}; A_5 = \sum_i p_0^2; A_6 = -\sum_i \frac{p_0^2}{I_i}$$

$$B_1 = \sum_i \frac{p_0^2}{p_i^2}(p_0 - p_i); B_2 = -\sum_i \frac{p_0}{p_i}(p_0 - p_i)\frac{1}{I_i}; B_3 = \sum_i \frac{p_0^2}{p_i}(p_0 - p_i); B_4 = p_0 \cdot B_2$$

fortschreitend mit der Erfassung der Wertepaare gebildet, wobei $p_0$ ein Referenzdruck, beispielsweise der Normdruck von 1013mbar, ist. Die Koeffizienten $A_1$ - $A_6$, $B_1$ - $B_4$ wurden zu diesem Zweck in einem elektronischen Speichermedium einer elektronischen Steuerungseinheit abgelegt und schritthaltend mit jedem weiteren erfassten Wertepaar $I_i$, $p_i$ durch Aufsummierung des jeweiligen Terms modifiziert. Eine dauerhafte Speicherung der Wertepaare $I_i$, $p_i$ erfolgte hingegen in ressourcenschonende Art und Weise nicht.

Anschließend wurde aus den Koeffizienten $A_1$ - $A_6$, $B_1$ - $B_4$ der Kompensationsparameter k bestimmt. Hierzu wurde die kubische Gleichung für den Kompilationsparameter k

$$(-A_1 \cdot B_2 + A_2 \cdot B_1) \cdot k^3 + (-A_1 \cdot B_2 \cdot p_0 + A_2 \cdot B_3 - A_3 \cdot B_2 + A_4 \cdot B_1) \cdot k^2$$
$$+ (-A_3 \cdot B_2 \cdot p_0 + A_4 \cdot B_3 - A_5 \cdot B_2 + A_6 \cdot B_1) \cdot k + (-A_5 \cdot B_2 \cdot p_0 + A_6 \cdot B_3) = 0$$

mit dem Newtonverfahren gelöst. Anschließend wurde der Skalierungsfaktor $m_{adap}$ gemäß der Formel

$$m_{adap} = -\frac{B_2(k + p_0)}{B_1 \cdot k + B_3}$$ berechnet.

Es ist in diesem Zusammenhang als sehr vorteilhaft hervorzuheben, dass die Speicherplatzbelegung zur Ermittlung des Kompensationsparameters k und des Skalierungsfaktor $m_{adap}$ unabhängig von der Anzahl der hierfür verwendeten Wertepaare $I_i$, $p_i$ ist.

In der anschließenden zweiten Betriebsart der Brennkraftmaschine wurden mit dem gleichen Gassensor fortlaufend Gaskonzentrationssignale $I_{nom}$ ermittelt. Das Abgasrückführungsventil war währenddessen in nicht fest vorgegebener Art und Weise entsprechend der Anforderung aus dem Betrieb der Brennkraftmaschine geöffnet, teilweise geöffnet und geschlossen.

Die Sauerstoffkonzentration in dem der Lambdasonde ausgesetzten Messgas, also der der Brennkraftmaschine zugeführten Luft schwankte entsprechend. Ferner schwankte das in der zweiten Betriebsart am Ort des Gassensors mittels eines Drucksensors ermittelte Drucksignal $p_{meas}$.

Ausgehend von dem in der zweiten Betriebsart gemessenen Gaskonzentrationssignals $I_{nom}$ wurde mittels der Formel

$$I(p_{meas}) = \frac{I_{nom}}{m_{adap}} \frac{p_{meas}}{k + p_{meas}} \cdot \frac{k + p_0}{p_0}$$

die tatsächliche Sauerstoffkonzentration stets mit hoher Genauigkeit bestimmt.

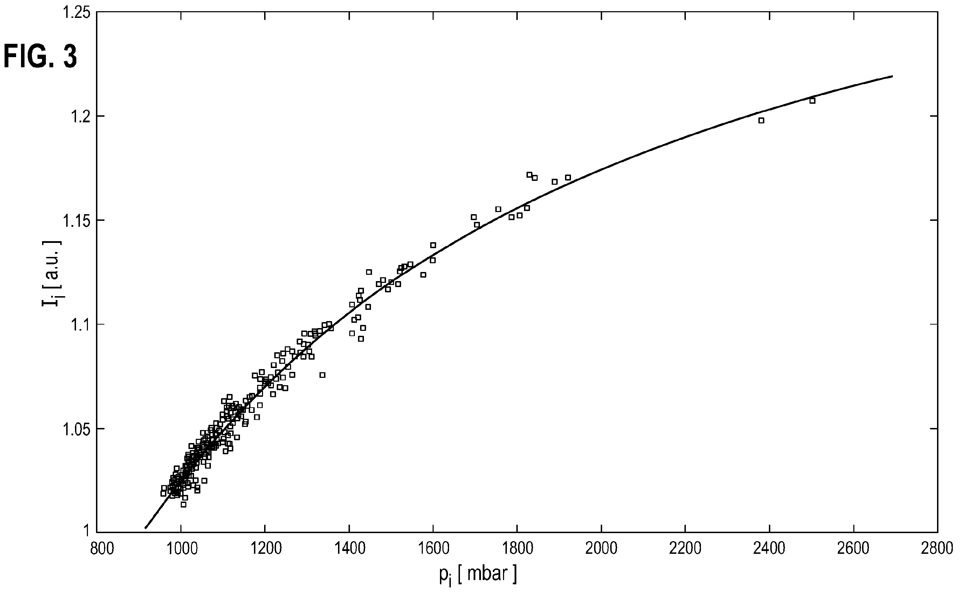

Figur 3 zeigt ein weiteres Beispiel erfindungsgemäß ermittelter Wertepaare $I_i$, $p_i$, mit denen das Verfahren ebenfalls durchgeführt werden kann. Die Anzahl der Wertepaare $I_i$, $p_i$ ist im Vergleich zu Figur 2 sehr viel größer. Der zur Bestimmung der tatsächlichen Sauerstoffkonzentration erforderliche Rechenaufwand nimmt jedoch auch mit den sehr vielen Wertepaare $I_i$, $p_i$ nur höchstens proportional zu der Anzahl der Wertepaare zu. Er ist somit bequem schritthaltend mit dem Anfallen der Wertepaare $I_i$, $p_i$ zu bewerkstelligen. Der im Steuergerät erforderliche Speicherplatz bleibt sogar unverändert.

## Patentansprüche

1. Verfahren zur Ermittlung einer Gaskonzentration in einem Messgas mit einem Gassensor (100), wobei in einer ersten Betriebsart einer Brennkraftmaschine, bei der die Gaskonzentration im Messgas bekannt ist, eine Vielzahl von Wertepaaren ($I_i$, $p_i$) jeweils eines Gaskonzentrationssignals ($I_i$) und eines Drucksignals ($p_i$) erfasst wird und ausgehend von diesen Wertepaaren ($I_i$, $p_i$) ein Kompensationsparameter (k) und ein Skalierungsfaktor ($m_{adap}$) des Gassensors (100) ermittelt werden, und wobei anschließend in einer zweiten Betriebsart der Brennkraftmaschine die Ermittlung einer zu bestimmenden Gaskonzentration erfolgt, auf Basis eines in der zweiten Betriebsart der Brennkraftmaschine gemessenen Gaskonzentrationssignals ($I_{nom}$) und unter Berücksichtigung des Kompensationsparameters (k) und des Skalierungsfaktor ($m_{adap}$) des Gassensors (100), **dadurch gekennzeichnet, dass** in der ersten Betriebsart der Kompensationsparameter (k) und der Skalierungsfaktor ($m_{adap}$) als Lösung von zwei Gleichungen bestimmt werden, wobei in die Gleichungen die Gaskonzentrationssignale ($I_i$) und dem Drucksignale ($p_i$) lediglich mittelbar über Koeffizienten ($A_1$ - $A_6$, $B_1$ - $B_3$) eingehen, die durch Summierung von von den Gaskonzentrationssignalen ($I_i$) und den Drucksignalen ($p_i$) abhängigen Termen gebildet werden.

2. Verfahren nach Anspruch 1, **dadurch gekennzeichnet, dass** in der ersten Betriebsart der Brennkraftmaschine ein Abgasrückführungsventil der Brennkraftmaschine geschlossen ist.

3. Verfahren nach Anspruch 1 oder 2, **dadurch gekennzeichnet, dass** in der zweiten Betriebsart der Brennkraftmaschine für die Ermittlung der Gaskonzentration der Wert einer zweiten vorgegebenen Funktion ($I(p_{meas})$) berechnet wird, die von dem in der zweiten Betriebsart gemessen Gaskonzentrationssignal ($I_{Nom}$) und dem in der zweiten Betriebsart ermittelten Drucksignal ($p_{meas}$) abhängig ist und deren Parameter der Kompensationsparameter (k) und der Skalierungsfaktor ($m_{adap}$) sind.

4. Verfahren nach Anspruch 1 oder 2, **dadurch gekennzeichnet, dass** in der ersten Betriebsart der Kompensationsparameter (k) und der Skalierungsfaktor ($m_{adap}$) als Lösung einer ersten Gleichung (G1):

$$(-A_1 \cdot B_2 + A_2 \cdot B_1) \cdot k^3 + (-A_1 \cdot B_2 \cdot p_0 + A_2 \cdot B_3 - A_3 \cdot B_2 + A_4 \cdot B_1) \cdot k^2$$
$$+ (-A_3 \cdot B_2 \cdot p_0 + A_4 \cdot B_3 - A_5 \cdot B_2 + A_6 \cdot B_1) \cdot k + (-A_5 \cdot B_2 \cdot p_0 + A_6 \cdot B_3) = 0$$

und einer zweiten Gleichung (G2):

$$m_{adap} = -\frac{B_2(k + p_0)}{B_1 \cdot k + B_3}$$

bestimmt werden, wobei $p_0$ ein Referenzdruck ($p_0$), k der Kompensationsparameter (k) und $m_{adap}$ der Skalierungsfaktor ($m_{adap}$) ist und wobei die Koeffizienten ($A_1$ - $A_6$, $B_1$ - $B_3$) berechnet werden gemäß:

$$A_1 = \sum_i \frac{p_0^2}{p_i^2}; A_2 = -\sum_i \frac{p_0}{p_i \cdot I_i}; A_3 = 2\sum_i \frac{p_0^2}{p_i}; A_4 = -\sum_i \frac{p_0}{p_i}(p_i + p_0)\frac{1}{I_i}; A_5 = \sum_i p_0^2; A_6 = -\sum_i \frac{p_0^2}{I_i}$$

$$B_1 = \sum_i \frac{p_0^2}{p_i^2}(p_0 - p_i); B_2 = -\sum_i \frac{p_0}{p_i}(p_0 - p_i)\frac{1}{I_i}; B_3 = \sum_i \frac{p_0^2}{p_i}(p_0 - p_i); B_4 = p_0 \cdot B_2$$

5. Verfahren nach dem vorangehenden Anspruch, **dadurch gekennzeichnet**, das die erste Gleichung (G1) nach dem Newton-Verfahren numerisch gelöst wird.

6. Verfahren nach einem der Ansprüche 4 oder 5, **dadurch gekennzeichnet, dass** in der zweiten Betriebsart der Brennkraftmaschine für die Ermittlung der Gaskonzentration die Größe $I(p_{meas})$ berechnet wird gemäß der Formel

$$I(p_{meas}) = \frac{I_{nom}}{m_{adap}} \frac{p_{meas}}{k + p_{meas}} \cdot \frac{k + p_0}{p_0}$$

wobei $I_{nom}$ das in der zweiten Betriebsart ermittelte Gaskonzentrationssignal ($I_{nom}$) und $p_0$ der Referenzdruck ($p_0$) und k der Kompensationsparameter (k) und $m_{adap}$ der Skalierungsfaktor ($m_{adap}$) ist.

7. Verfahren nach einem der vorhergehenden Ansprüche, **dadurch gekennzeichnet, dass** in der zweiten Betriebsart der Brennkraftmaschine die Gaskonzentration im Ansaugtrakt der Brennkraftmaschine, stromabwärts des bzw. eines Abgasrückführungsventils ermittelt wird.

8. Computerprogramm, umfassend Befehle, die bewirken, dass eine Brennkraftmaschine mit einem Gassensor und einer elektronischen Steuerungseinheit, welche geeignet ist, um die Schritte des Verfahrens nach einem der vorangehenden Ansprüche durchzuführen, die Schritte des Verfahrens nach einem der vorangehenden Ansprüche durchführt.

9. Elektronisches Speichermedium, auf welchem ein Computerprogramm nach dem vorangehenden Anspruch gespeichert ist.

10. Elektronische Steuerungseinheit, welches ein elektronisches Speichermedium nach dem vorangehenden Anspruch umfasst.

11. Elektronische Steuerungseinheit nach Anspruch 10, **dadurch gekennzeichnet, dass** die Koeffizienten ($A_1$ - $A_6$, $B_1$ - $B_3$) in der Steuerungseinheit, insbesondere in dem elektronischen Speichermedium, gespeichert sind.

**Claims**

1. Method for determining a gas concentration in a measurement gas using a gas sensor (100), a multiplicity of value pairs ($I_i$, $p_i$) of respectively a gas concentration signal ($I_i$) and a pressure signal ($p_i$) being registered in a first mode of operation of an internal combustion engine, in which the gas concentration in the measurement gas is known, and a compensation parameter (k) and a scaling factor ($m_{adap}$) of the gas sensor (100) being determined using these value pairs ($I_i$, $p_i$) as a starting point,
and a gas concentration to be determined subsequently being determined in a second mode of operation of the internal combustion engine, on the basis of a gas concentration signal ($I_{nom}$) measured in the second mode of operation of the internal combustion engine and with the compensation parameter (k) and the scaling factor ($m_{adap}$) of the gas sensor (100) being taken into account, **characterized in that** the compensation parameter (k) and the scaling factor ($m_{adap}$) are determined in the first mode of operation as the solution of two equations, the gas concentration signals ($I_i$) and the pressure signals ($p_i$) only being included indirectly in the equations by way of coefficients ($A_1$-$A_6$, $B_1$-$B_3$) which are formed by the summation of terms that are dependent on the gas concentration signals ($I_i$) and on the pressure signals ($p_i$).

2. Method according to Claim 1, **characterized in that** an exhaust gas recirculation valve of the internal combustion engine is closed in the first mode of operation of the internal combustion engine.

3. Method according to Claim 1 or 2, **characterized in that**, in the second mode of operation of the internal combustion engine, the value of a second specified function ($I(p_{meas})$) is calculated for the determination of the gas concentration, the second specified function depending on the gas concentration signal ($I_{Nom}$) measured in the second mode of operation and on the pressure signal ($p_{meas}$) determined in the second mode of operation and the parameters of said second specified function being the compensation parameter (k) and the scaling factor ($m_{adap}$).

4. Method according to Claim 1 or 2, **characterized in that**, in the first mode of operation, the compensation parameter (k) and the scaling factor ($m_{adap}$) are determined as solution to a first equation (G1):

$$(-A_1 \cdot B_2 + A_2 \cdot B_1) \cdot k^3 + (-A_1 \cdot B_2 \cdot p_0 + A_2 \cdot B_3 - A_3 \cdot B_2 + A_4 \cdot B_1) \cdot k^2$$
$$+ (-A_3 \cdot B_2 \cdot p_0 + A_4 \cdot B_3 - A_5 \cdot B_2 + A_6 \cdot B_1) \cdot k + (-A_5 \cdot B_2 \cdot p_0 + A_6 \cdot B_3) = 0$$

and a second equation (G2):

$$m_{adap} = -\frac{B_2(k + p_0)}{B_1 \cdot k + B_3}$$

where $p_0$ is a reference pressure ($p_0$), k is the compensation parameter (k) and $m_{adap}$ is the scaling factor ($m_{adap}$), and the coefficients ($A_1$-$A_6$, $B_1$-$B_3$) are calculated according to:

$$A_1 = \sum_i \frac{p_0^2}{p_i^2}; A_2 = -\sum_i \frac{p_0^2}{p_i \cdot I_i}; A_3 = 2\sum_i \frac{p_0^2}{p_i}; A_4 = -\sum_i \frac{p_0}{p_i}(p_i + p_0)\frac{1}{I_i}; A_5 = \sum_i p_0^2; A_6 = -\sum_i \frac{p_0^2}{I_i}$$

$$B_1 = \sum_i \frac{p_0^2}{p_i^2}(p_0 - p_i); B_2 = -\sum_i \frac{p_0}{p_i}(p_0 - p_i)\frac{1}{I_i}; B_3 = \sum_i \frac{p_0^2}{p_i}(p_0 - p_i); B_4 = p_0 \cdot B_2$$

5. Method according to the preceding claim, **characterized in that** the first equation (G1) is solved numerically using the Newton method.

6. Method according to either of Claims 4 and 5, **characterized in that**, in the second mode of operation of the internal combustion engine, the quantity I ($p_{meas}$) is calculated for the determination of the gas concentration, the calculation being carried out according to the formula

$$I(p_{meas}) = \frac{I_{nom}}{m_{adap}} \frac{p_{meas}}{k + p_{meas}} \cdot \frac{k + p_0}{p_0}$$

where $I_{nom}$ is the gas concentration signal ($I_{nom}$) determined in the second mode of operation and $p_0$ is the reference pressure ($p_0$) and k is the compensation parameter (k) and $m_{adap}$ is the scaling factor ($m_{adap}$).

7. Method according to any one of the preceding claims, **characterized in that**, in the second mode of operation of the internal combustion engine, the gas concentration is determined in the induction tract of the internal combustion engine, downstream of the or an exhaust gas recirculation valve.

8. Computer program comprising commands which cause an internal combustion engine with a gas sensor and an electronic control unit suitable for carrying out the steps of the method according to any one of the preceding claims to carry out the steps of the method according to any one of the preceding claims.

9. Electronic storage medium on which a computer program according to the preceding claim is stored.

10. Electronic control unit which comprises an electronic storage medium according to the preceding claim.

11. Electronic control unit according to Claim 10, **characterized in that** the coefficients ($A_1$-$A_6$, $B_1$-$B_3$) are stored in the control unit, more particularly in the electronic storage medium.

**Revendications**

1. Procédé de détermination d'une concentration de gaz dans un gaz de mesure au moyen d'un capteur de gaz (100), dans lequel, dans un premier mode de fonctionnement d'un moteur à combustion interne, dans lequel la concentration de gaz dans le gaz de mesure est connue, une pluralité de paires de valeurs ($I_i$, $p_i$) respectivement d'un signal de concentration de gaz ($I_i$) et d'un signal de pression ($p_i$) est détectée, et à partir de ces paires de valeurs ($I_i$, $p_i$), un paramètre de compensation (k) et un facteur d'échelle ($m_{adap}$) du capteur de gaz (100) sont déterminés,

   et dans lequel ensuite, dans un deuxième mode de fonctionnement du moteur à combustion interne, la détermination d'une concentration de gaz à spécifier est effectuée sur la base d'un signal de concentration de gaz ($I_{nom}$) mesuré dans le deuxième mode de fonctionnement du moteur à combustion interne et en tenant compte du paramètre de compensation (k) et du facteur d'échelle ($m_{adap}$) du capteur de gaz (100), **caractérisé en ce que** dans le premier mode de fonctionnement, le paramètre de compensation (k) et le facteur d'échelle ($m_{adap}$) sont spécifiés comme solution de deux équations, dans lequel, les signaux de concentration de gaz ($I_i$) et les signaux de pression ($p_i$) n'entrent dans les équations qu'indirectement par l'intermédiaire de coefficients ($A_1$ à $A_6$, $B_1$ à $B_3$) qui sont formés par la sommation de termes dépendant des signaux de concentration de gaz ($I_i$) et des signaux de pression ($p_i$).

2. Procédé selon la revendication 1, **caractérisé en ce que** dans le premier mode de fonctionnement du moteur à combustion interne, une soupape de recirculation des gaz d'échappement du moteur à combustion interne est fermée.

3. Procédé selon la revendication 1 ou 2, **caractérisé en ce que** dans le deuxième mode de fonctionnement du moteur à combustion interne, pour la détermination de la concentration de gaz, la valeur d'une deuxième fonction prédéfinie ($I(p_{meas})$) est calculée qui dépend du signal de concentration de gaz ($I_{nom}$) mesuré dans le deuxième mode de fonctionnement et du signal de pression ($p_{meas}$) déterminé dans le deuxième mode de fonctionnement, et dont les paramètres sont le paramètre de compensation (k) et le facteur d'échelle ($m_{adap}$).

4. Procédé selon la revendication 1 ou 2, **caractérisé en ce que** dans le premier mode de fonctionnement, le paramètre de compensation (k) et le facteur d'échelle ($m_{adap}$) sont spécifiés comme solution d'une première équation (G1) :

$$(-A_1 \cdot B_2 + A_2 \cdot B_1) \cdot k^3 + (-A_1 \cdot B_2 \cdot p_0 + A_2 \cdot B_3 - A_3 \cdot B_2 + A_4 \cdot B_1) \cdot k^2$$
$$+ (-A_3 \cdot B_2 \cdot p_0 + A_4 \cdot B_3 - A_5 \cdot B_2 + A_6 \cdot B_1) \cdot k + (-A_5 \cdot B_2 \cdot p_0 + A_6 \cdot B_3) = 0$$

et d'une deuxième équation (G2) :

$$m_{adap} = -\frac{B_2(k + p_0)}{B_1 \cdot k + B_3}$$

où $p_0$ est une pression de référence ($p_0$), k est le paramètre de compensation (k), et $m_{adap}$ est le facteur d'échelle ($m_{adap}$), et les coefficients ($A_1$ à $A_6$, $B_1$ à $B_3$) étant calculés selon :

$$A_1 = \sum_i \frac{p_0^2}{p_i^2} \; ; \; A_2 = -\sum_i \frac{p_0}{p_i \cdot I_i} \; ; \; A_3 = 2\sum_i \frac{p_0^2}{p_i} \; ; \; A_4 = -\sum_i \frac{p_0}{p_i}(p_i + p_0)\frac{1}{I_i} \; ;$$

$$A_5 = \sum_i p_0^2 \; ; \; A_6 = -\sum_i \frac{p_0^2}{I_i}$$

$$B_1 = \sum_i \frac{p_0^2}{p_i^2}(p_0 - p_i) \; ; \; B_2 = -\sum_i \frac{p_0}{p_i}(p_0 - p_i)\frac{1}{I_i} \; ; \; B_3 = \sum_i \frac{p_0^2}{p_i}(p_0 - p_i) \; ;$$

$$B_4 = p_0 \cdot B_2$$

**5.** Procédé selon la revendication précédente, **caractérisé en ce que** la première équation (G1) est résolue sous forme numérique selon la méthode de Newton.

**6.** Procédé selon l'une quelconque des revendications 4 ou 5, **caractérisé en ce que** dans le deuxième mode de fonctionnement du moteur à combustion interne, pour la détermination de la concentration de gaz, la grandeur I ($p_{meas}$) est calculée selon la formule

$$I(p_{meas}) = \frac{I_{nom}}{m_{adap}}\frac{p_{meas}}{k + p_{meas}} \cdot \frac{k + p_0}{p_0}$$

où $I_{nom}$ est le signal de concentration de gaz ($I_{nom}$) déterminé dans le deuxième mode de fonctionnement, et $p_0$ est la pression de référence ($p_0$), et k est le paramètre de compensation (k) et $m_{adap}$ est le facteur d'échelle ($m_{adap}$).

**7.** Procédé selon l'une quelconque des revendications précédentes, **caractérisé en ce que** dans le deuxième mode de fonctionnement du moteur à combustion interne, la concentration de gaz dans la conduite d'admission du moteur à combustion interne, est déterminée en aval de la ou d'une soupape de recirculation des gaz d'échappement.

**8.** Programme informatique, comprenant des commandes qui font qu'un moteur à combustion interne comprenant un capteur de gaz et une unité de commande électronique qui est apte à exécuter les étapes du procédé selon l'une quelconque des revendications précédentes, exécute les étapes du procédé selon l'une quelconque des revendications précédentes.

**9.** Support de stockage électronique sur lequel est stocké un programme informatique selon la revendication précédente.

**10.** Unité de commande électronique qui comprend un support de stockage électronique selon la revendication précédente.

**11.** Unité de commande électronique selon la revendication 10, **caractérisée en ce que** les coefficients ($A_1$ à $A_6$, $B_1$ à $B_3$) sont stockés sur l'unité de commande, en particulier sur le support de stockage électronique.

EP 3 368 759 B1

# FIG. 1

# FIG. 2

EP 3 368 759 B1

EP 3 368 759 B1

**FIG. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006011837 A1
- US 2015267627 A1
- US 20140150760 A1